# EUROPEAN PATENT APPLICATION

(11) **EP 3 741 838 A1**
(43) Date of publication of application: **25.11.2020**
(21) Application number: 18900467.4
(22) Date of filing: 17.12.2018
(51) Int. Cl.: C12M 1/00, C12M 3/00, C12M 3/02, C12M 3/06, C12M 3/08

(54) **CELL CULTURE DEVICE AND CELL CULTURE METHOD**

(30) Priority: 15.01.2018 JP 2018004222
(71) Applicant: Fujifilm Corporation, Minato-ku Tokyo 106-8620 (JP)
(72) Inventor: INADA, Atsushi, Ashigarakami-gun, Kanagawa 258-8577 (JP); TAKAYAMA, Hidetoshi, Ashigarakami-gun, Kanagawa 258-8577 (JP)
(74) Representative: Klunker IP Patentanwälte PartG mbB
(86) International application number: PCT/JP2018/046422
(87) International publication number: WO 2019/138796

(57) **Abstract**

A cell culture device includes a plurality of culture containers that divide and accommodate a cell suspension; a treatment part that performs a treatment on a certain amount, which corresponds to each of units of treatment, of at least one of a cell suspension accommodated in a culture container, or cells contained in the cell suspension accommodated in the culture container; a mixing part that mixes cell suspensions which are treated in the treatment part and which respectively correspond to each of the plurality of units of treatment; a stirring part that stirs the cell suspension mixed in the mixing part; and a transfer control unit that controls a transfer of the cell suspension to the treatment part, the mixing part, and the stirring part.

## Description

### BACKGROUND OF THE INVENTION

### 1. Field of the Invention

The disclosed technology relates to a cell culture device and a cell culture method.

### 2. Description of the Related Art

The following technologies are known as technologies relating to a cell culture device. For example, JP2005-198626A discloses a device for subculturing adherent cells, in which a plurality of culture containers are disposed, one common treatment container is connected to these culture containers, and a valve unit is interposed between each of the culture containers and the treatment container, where the treatment container has a filter unit through which a medium, and a washing solution and peeling solution are able to pass and through which cells are prevented from passing, and the treatment container has a discharge outlet for discharging the medium, and the washing solution and peeling solution which have passed through the filter unit to the outside.

In addition, JP2016-131538A discloses a cell culture device including: a culture container for culturing cells; a reserving container for reserving the cells cultured in the culture container; and a dividing treatment part for performing a dividing treatment for dividing a cell mass.

### SUMMARY OF THE INVENTION

A cell culture device that automates at least part of a treatment required in cell culture is configured by including, for example, a culture container that accommodates a cell suspension, a treatment part that performs a predetermined treatment on the cell suspension accommodated in the culture container or cells contained in the cell suspension, and a transfer control unit that controls a transfer of the cell suspension from the culture container to the treatment part. In a case where a mass culture of culturing, for example, 10¹⁰ or more cells is performed using the above-mentioned cell culture device having such a configuration, a method is conceivable in which the cell suspension is divided into a plurality of culture containers, and in this divided state, the cell suspensions are accommodated in an incubator to be cultured. A predetermined amount of the cell suspensions divided and accommodated in the plurality of culture containers is extracted from the culture container according to a treatment capacity of the treatment part, and is sequentially transferred to the treatment part. The treatment part performs a treatment on the cell suspension transferred in a stepwise manner from the culture container in the order of transfer.

It is assumed that, for example, cells produced using the above-mentioned cell culture device are accommodated in recovery containers at a predetermined amount to be shipped as a product. For example, in a case where produced cells are used for regenerative medicine, it is required that quality of cells between the recovery containers be uniform. However, in a case of dividing cell suspensions into a plurality of culture containers to culture cells, there is a concern that quality of cells becomes different between culture containers due to, for example, a slight temperature difference in an incubator, and the like. Furthermore, according to the cell culture device having the above configuration, there is a concern that treatment states in the treatment part change in accordance with an increase in an amount of cell suspensions in cumulative treatment or the number of cells in cumulative treatment in the treatment part. For example, in a case where the treatment part includes a filtration device that has a filtration membrane and performs a membrane separation treatment on the cell suspensions, there is a concern that debris such as dead cells accumulates on the filtration membrane in accordance with an increase in an amount of cell suspensions in cumulative treatment in the filtration device, and thus a filtration performance of the filtration membrane changes. In a case where treatment states in the treatment part change, there is a concern that quality of treated cells changes. That is, there is a concern that quality of cells that have been treated earlier in the treatment part becomes different from quality of cells that have been treated later.

An object of the disclosed technology is to absorb differences in culture states between culture containers in a case where a cell suspension is divided into a plurality of culture containers to perform cell culture.

A cell culture device of the disclosed technology comprises: a plurality of culture containers that divide and accommodate a cell suspension; a treatment part that performs a treatment on a certain amount, which corresponds to each of units of treatment, of at least one of a cell suspension accommodated in each of the plurality of culture containers, or cells contained in the cell suspension accommodated in each of the plurality of culture containers; a mixing part that mixes cell suspensions which are treated in the treatment part and which respectively correspond to each of the plurality of units of treatment; a stirring part that stirs the cell suspension mixed in the mixing part; and a transfer control unit that controls a transfer of the cell suspension to the treatment part, the mixing part, and the stirring part. According to the cell culture device according to the disclosed technology, it is possible to absorb differences in culture states between culture containers in a case where a cell suspension is divided into a plurality of culture containers to perform cell culture.

The cell culture device according to the disclosed technology may further comprise a temperature control unit that controls a temperature of the cell suspension mixed in the mixing part. According to this aspect, it is possible to prevent the cell suspension from being exposed to a temperature environment that is not suitable for culturing cells for a long time, and thereby a survival rate of cells can be increased.

In the cell culture device according to the disclosed technology, the mixing part may be configured integrally with the stirring part. According to this aspect, a reduction in size of the device can be realized. In addition, because it is not necessary to transfer the cell suspension between the mixing part and the stirring part, damage to cells due to a transfer of the cell suspension can be reduced.

In the cell culture device according to the disclosed technology, the mixing part preferably mixes the cell suspensions which are treated in the treatment part and which respectively correspond to each of the plurality of units of treatment. According to this aspect, an effect of inhibiting variations in quality of cells associated with a temporal fluctuation in treatment states in the treatment part is promoted.

The cell culture device according to the disclosed technology may further comprise a plurality of recovery containers that recover the cell suspension stirred in the stirring part. In this case, the transfer control unit further controls a transfer of the cell suspension to each of the plurality of recovery containers. According to this aspect, a state is provided in which quality of cells is substantially uniform between the recovery containers.

In the cell culture device according to the disclosed technology, the transfer control unit may further control a transfer of the cell suspension stirred in the stirring part to each of the plurality of culture containers. According to this aspect, it becomes possible to reuse the plurality of culture containers for cells to continue cell culture.

In the cell culture device according to the disclosed technology, the mixing part may include a plurality of reserving parts that reserves the cell suspensions treated in the treatment part or the cell suspensions containing the cells treated in the treatment part for each of the units of treatment in the treatment part, and a merging part that merges the cell suspensions respectively extracted by a predetermined amount from each of the plurality of reserving parts. According to this aspect, because the stirring part is not required to have a volume capable of accommodating a total amount of the cell suspensions accommodated in the plurality of culture containers, it is possible to reduce a volume of the stirring part.

In the cell culture device according to the disclosed technology, the cell suspensions, which are treated in the treatment part and respectively correspond to each of the plurality of units of treatment, are preferably respectively reserved in any of the plurality of reserving parts. According to this aspect, an effect of inhibiting variations in quality of cells associated with a temporal fluctuation in treatment states in the treatment part is promoted.

A cell culture method according to the disclosed technology comprises: performing a treatment on a certain amount, which corresponds to each of units of treatment, of at least one of a cell suspension or cells contained in the cell suspension; mixing treated cell suspensions which respectively correspond to each of the plurality of units of treatment; and stirring the mixed cell suspension. According to the cell culture method according to the disclosed technology, it is possible to absorb differences in culture states between culture containers in a case where a cell suspension is divided into a plurality of culture containers to perform cell culture.

According to the disclosed technology, it is possible to inhibit variations in quality of cells associated with a temporal fluctuation in treatment states in the treatment part.

### BRIEF DESCRIPTION OF THE DRAWINGS

Fig. 1 is a diagram illustrating an example of a configuration of a cell culture device according to a first embodiment of the disclosed technology.
Fig. 2 is a diagram illustrating an example of a configuration of a treatment part according to the embodiment of the disclosed technology.
Fig. 3 is a diagram illustrating another example of a configuration of the treatment part according to the embodiment of the disclosed technology.
Fig. 4 is a flowchart illustrating an example of an operation sequence of a transfer control unit according to the embodiment of the disclosed technology.
Fig. 5A is a diagram illustrating an example of a state in which a cell suspension corresponding to each unit of treatment is accommodated in a mixing part according to the embodiment of the disclosed technology.
Fig. 5B is a diagram illustrating an example of a state in which a cell suspension corresponding to each unit of treatment is transferred to a stirring part according to the embodiment of the disclosed technology.
Fig. 5C is a diagram illustrating an example of a state in which, by a batch treatment, a cell suspension is accommodated in a plurality of recovery containers according to the embodiment of the disclosed technology.
Fig. 6 is a diagram illustrating an example of a configuration of a cell culture device according to a second embodiment of the disclosed technology.
Fig. 7 is a diagram illustrating an example of a configuration of a cell culture device according to a third embodiment of the disclosed technology.
Fig. 8 is a diagram illustrating an example of a configuration of a cell culture device according to a fourth embodiment of the disclosed technology.
Fig. 9 is a diagram illustrating an example of a configuration of a mixing part according to the fourth embodiment of the disclosed technology.
Fig. 10 is a flowchart illustrating an example of an operation sequence of a transfer control unit according to the fourth embodiment of the disclosed technology.

### DESCRIPTION OF THE PREFERRED EMBODIMENTS

Hereinafter, an example of an embodiment of the present invention will be described with reference to the drawings. In the respective drawings, the same or equivalent components and portions are denoted by the same reference numerals, and redundant description will be omitted as appropriate.

### [First embodiment]

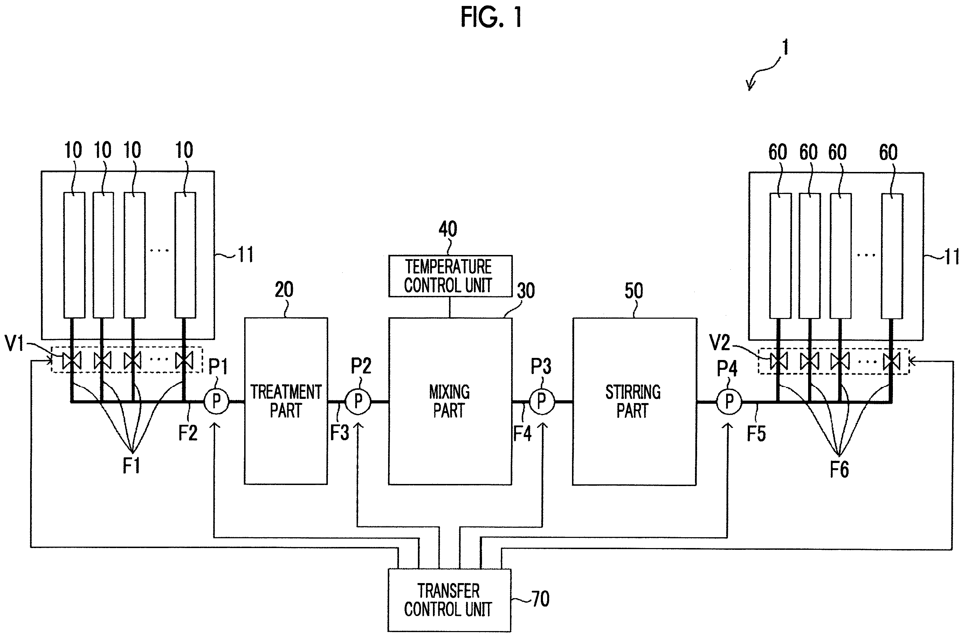

Fig. 1 is a diagram illustrating an example of a configuration of a cell culture device 1 according to a first embodiment of the disclosed technology. The cell culture device 1 includes a plurality of culture containers 10, a treatment part 20, a mixing part 30, a temperature control unit 40, a stirring part 50, a plurality of recovery containers 60, and a transfer control unit 70.

The plurality of culture containers 10 are containers that divide and accommodate a cell suspension containing cells to be cultured. Each of the plurality of culture containers 10 may have a form of a bag consisting of a gas-permeable film. A total volume of the plurality of culture containers 10 is, for example, a volume that can accommodate 10¹⁰ or more cells together with a medium. By dividing and accommodating a large amount of a cell suspension containing 10¹⁰ or more cells in the plurality of culture containers 10, a volume of each of the culture containers 10 can be reduced. Thereby, in each of the culture containers 10, it becomes possible to maintain a favorable supply state of oxygen, which is introduced into the culture container 10 through a surface of the culture container 10, to each cell.

The plurality of culture containers 10 are accommodated inside an incubator 11. A temperature inside the incubator 11 is maintained at a temperature (for example, 37°C) suitable for culturing cells accommodated in the culture containers 10.

Each of the plurality of culture containers 10 is connected to the treatment part 20 via individual flow paths F1 and a flow path F2. Each of the individual flow paths F1 has a valve V1. Each of the individual flow paths is connected to the flow path F2, and a pump P1 is provided on the flow path F2. The pump P1 is driven in a case of transferring cell suspensions accommodated in each of the culture containers 10 to the treatment part 20.

The treatment part 20 performs a predetermined treatment on at least one of a cell suspension accommodated in each of the culture containers 10 or cells contained in the cell suspension accommodated in each of the culture containers 10, at each predetermined unit of treatment. That is, the treatment part 20 does not collectively perform a treatment on a total amount of the cell suspensions respectively accommodated in each of the plurality of culture containers 10, but performs a treatment on a treatment amount thereof according to a treatment capacity of the treatment part 20 as one unit of treatment. The treatment performed in the treatment part 20 is not particularly limited, and examples thereof may include a membrane separation treatment of a cell suspension, or may include a dividing treatment that divides an aggregate (spheres) formed by aggregation of a plurality of cells into a small-scale aggregate having a smaller number of cells.

The treatment part 20 is connected to the mixing part 30 via a flow path F3. A pump P2 is provided on the flow path F3. The pump P2 is driven in a case if transferring the cell suspensions treated in the treatment part 20 to the mixing part 30.

The mixing part 30 has a form of a container having a volume capable of accommodating a total amount of the cell suspensions accommodated in each of the plurality of culture containers 10, and mixes the cell suspensions which are treated in the treatment part 20 and respectively correspond to each of a plurality of units of treatment. That is, it is a state in which a cell suspension that has been treated in the treatment part 20 is sequentially transferred to the mixing part 30 at each unit of treatment, and the cell suspensions respectively corresponding to each of the plurality of units of treatment are mixed in the mixing part 30.

The temperature control unit 40 controls a temperature of the cell suspension mixed in the mixing part 30 to a temperature suitable for culturing cells (for example, 37°C). The temperature control unit 40 may include, for example, a heater and a cooler that control atmospheric temperature within the mixing part 30. For example, the temperature control unit 40 may control the temperature of the cell suspension based on a temperature detection signal from a temperature sensor (not shown) that detects a temperature of the cell suspension accommodated in the mixing part 30.

The mixing part 30 is connected to the stirring part 50 via a flow path F4. A pump P3 is provided on the flow path F4. The pump P3 is driven in a case of transferring the cell suspension mixed in the mixing part 30 to the stirring part 50.

The stirring part 50 stirs the cell suspension mixed in the mixing part 30. That is, the stirring part 50 stirs the mixture in which the cell suspensions respectively corresponding to each of the plurality of units of treatment are mixed. In a case of sending a solution of cells mixed in the mixing part 30 to the recovery containers 60, the stirring part 50 performs stirring for the purpose of equalizing a density of cells and cell aggregates contained in the cell suspension and a size distribution of the cell aggregates, and equalizing quality of cells between the recovery containers 60. A stirring method for the stirring part 50 is not particularly limited, and for example, it is possible to apply a method using a stirring blade, a siphon method, a vertical stirring method, and the like.

The plurality of recovery containers 60 are containers that divide and accommodate the cell suspension stirred in the stirring part 50. A form of the recovery containers 60 may be the same as that of the culture containers 10. A total volume of the plurality of recovery containers 60 may be equal to or greater than a total volume of the plurality of culture containers 10. In addition, a total volume of the plurality of recovery containers 60 may be smaller than a total volume of the plurality of culture containers 10. Each of the recovery containers 60 is accommodated inside the incubator 11 in which the culture containers 10 are accommodated. Each of the recovery containers 60 may be accommodated inside a freezer for freezing and storing a cell suspension.

Each of the recovery containers 60 is connected to the stirring part 50 via individual flow paths F6 and a flow path F5. Each of the individual flow paths F6 has a valve V2. Each of the individual flow paths F6 is connected to the flow path F5, and a pump P4 is provided on the flow path F5. The pump P4 is driven in a case of transferring the cell suspension stirred in the stirring part 50 to the recovery containers 60.

The transfer control unit 70 controls each of a transfer of the cell suspension from the culture containers 10 to the treatment part 20, a transfer of the cell suspension from the treatment part 20 to the mixing part 30, a transfer of the cell suspension from the mixing part 30 to the stirring part 50, and a transfer of the cell suspension from the stirring part 50 to the recovery containers 60. The transfer control unit 70 controls transfers of the cell suspension by controlling opening and closing of the valves V1 and V2 and by controlling driving of the pumps P1, P2, P3, and P4.

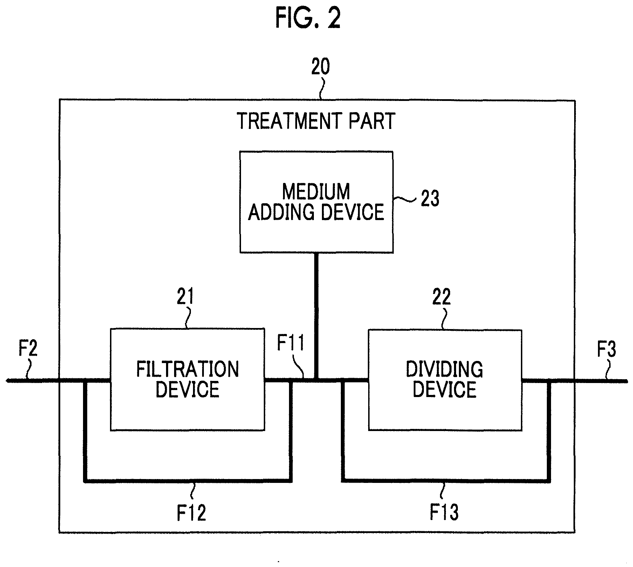

Fig. 2 is a diagram illustrating an example of a configuration of the treatment part 20. Fig. 2 does not illustrate a solution sending unit such as a pump for sending the cell suspension, and a path regulating unit such as a valve for regulating a path through which the cell suspension passes. The treatment part 20 is configured by including, for example, a filtration device 21, a dividing device 22, and a medium adding device 23.

The filtration device 21 includes a filtration membrane (not shown), and performs a membrane separation treatment (concentration treatment) on the cell suspension supplied through the flow path F2. The membrane separation treatment (concentration treatment) is a treatment of separating cells contained in a cell suspension and debris containing a used medium, dead cells, and the like by a filtration membrane. The cell suspension concentrated by passing through the filtration device 21 is discharged to a flow path F11.

### A bypass flow path F12 has one end connected to the flow path F2 and the other end connected to the flow path F11. The bypass flow path F12 is used in a case where the cell suspension flowing into the treatment part 20 via the flow path F2 is transferred to the dividing device 22 without passing through the filtration device 21. Furthermore, the bypass flow path F12 can be used also in a case where the membrane separation treatment is again performed by the filtration device 21 on the cell suspension discharged into the flow path F11.

The medium adding device 23 supplies a fresh medium to the cell suspension concentrated by the membrane separation treatment (concentration treatment) by the filtration device 21. By cooperation of the filtration device 21 and the medium adding device 23, it is possible to perform a medium exchange for replacing a used medium with a fresh medium.

The dividing device 22 performs a dividing treatment of dividing an aggregate (sphere) in which a plurality of cells contained in the cell suspension are aggregated into a small-scale aggregate having a smaller number of cells. The dividing device 22 includes a mesh having a plurality of meshes formed by knitting a fibrous member. A cell mass larger than a mesh size of the mesh passes through the mesh and is divided into cell masses having a size corresponding to the mesh size of the mesh.

For example, in culture of pluripotent stem cells, in a case where a size of cell aggregates generated by culturing the cells becomes excessively large, there is a problem of the aggregates adhering and fusing with each other, the cells starting to differentiate, or the center of the aggregates causing necrosis of cells. Accordingly, to prevent a size of the aggregates from becoming excessively large, it is necessary to perform a dividing treatment of dividing (disintegrating) the aggregates at an appropriate time during a culture period. The cell mass divided by passing through the dividing device 22 is discharged into the flow path F3.

A bypass flow path F13 has one end connected to the flow path F11 and the other end connected to the flow path F3. The bypass flow path F13 is used, for example, in a case of transferring the cell suspension subjected to the membrane separation treatment by the filtration device 21 to the mixing part 30 without going through the dividing device 22. In addition, the bypass flow paths F12 and F13 can be used in a case where the membrane separation treatment is performed by the filtration device 21 after the dividing treatment is performed by the dividing device 22.

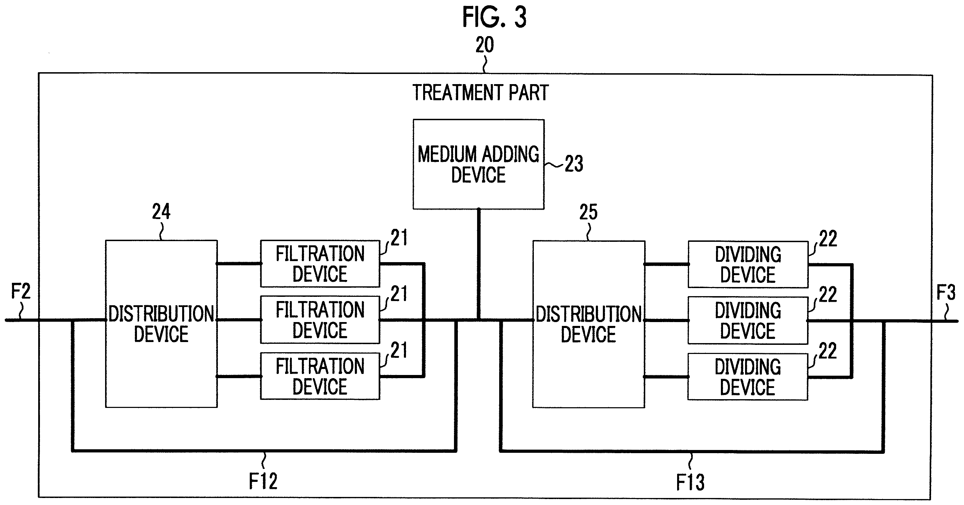

Fig. 3 is a diagram illustrating another example of a configuration of the treatment part 20. As illustrated in Fig. 3, the treatment part 20 may be configured by including a plurality of filtration devices 21, a plurality of dividing devices 22, and distribution devices 24 and 25.

The distribution device 24 distributes the cell suspension flowing into the treatment part 20 via the flow path F2 to each of the plurality of filtration devices 21. Each of the plurality of filtration devices 21 performs a membrane separation treatment (concentration treatment) on the cell suspension distributed by the distribution device 24. The membrane separation treatment (concentration treatment) can be parallelized by cooperation of the distribution device 24 and the plurality of filtration devices 21, whereby a treatment time of the membrane separation treatment (concentration treatment) can be shortened.

The distribution device 25 distributes the cell suspension discharged from the filtration device 21 or the cell suspension supplied via the bypass flow path F12 to each of the plurality of dividing devices 22. Each of the plurality of dividing devices 22 performs a dividing treatment on the cells contained in the cell suspension distributed by the distribution device 25. By cooperation of the distribution device 25 and the plurality of dividing devices 22, the dividing treatment can be parallelized, and thereby a treatment time for the dividing treatment can be shortened.

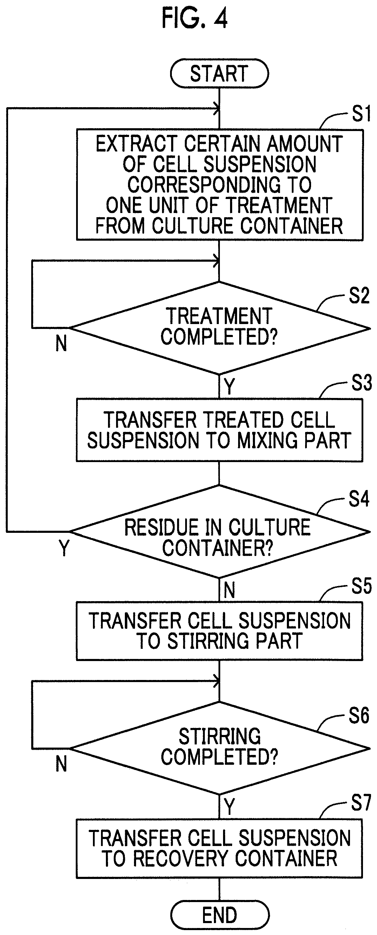

Hereinafter, operation of the cell culture device 1 will be described with reference to Fig. 4. Fig. 4 is a flowchart illustrating an example of an operation sequence of the transfer control unit 70. In an initial state, it is assumed that the cell suspension containing the cells to be cultured is accommodated in each of the plurality of culture containers 10 disposed in the incubator 11.

In Step S1, the transfer control unit 70 controls one of the valves V1 to be selectively opened and drives the pump P1. That is, the transfer control unit 70 extracts a certain amount of the cell suspension corresponding to one unit of treatment in the treatment part 20 among the cell suspensions accommodated in each of the plurality of culture containers 10 from any of the culture containers 10. In this case, a part of the cell suspensions may be extracted from each of the culture containers 10, or a part or all of the cell suspensions may be extracted from at least one of the culture containers 10. The cell suspension extracted from the culture container 10 is transferred to the treatment part 20 and is subjected to a predetermined treatment in the treatment part 20.

In Step S2, the transfer control unit 70 determines whether the treatment in the treatment part 20 is completed. The transfer control unit 70 may determine that the treatment in the treatment part 20 is completed based on, for example, a state in which a predetermined time has elapsed since the transfer of the cell suspension to the treatment part 20. In addition, in a case where a signal for notifying the completion of treatment is supplied from the treatment part 20, the transfer control unit 70 may determine that the treatment is completed in the treatment part 20 based on the signal. In a case where the transfer control unit 70 determines that the treatment has been completed in the treatment part 20, the treatment proceeds to Step S3.

In Step S3, the transfer control unit 70 drives the pump P2 to transfer a certain amount of the cell suspension that corresponds to one unit of treatment and has been treated in the treatment part 20 to the mixing part 30.

In Step S4, the transfer control unit 70 determines whether the cell suspension remains in any of the culture containers 10. The transfer control unit 70 may determine whether or not the cell suspension remains in the culture container 10 by, for example, determining whether an accumulated amount of the cell suspension extracted from the culture container 10 has reached a total amount of the cell suspension accommodated in each of the culture containers 10 in the initial state. In addition, in a case where information indicating a weight of the cell suspension accommodated in the culture container 10 is provided in real time, the transfer control unit 70 may determine whether or not the cell suspension remains in the culture container 10 based on the information.

In a case where the transfer control unit 70 determines that the cell suspension remains in any of the culture containers 10, the transfer control unit 70 returns the treatment to Step S1. That is, the transfer control unit 70 repeatedly performs the treatment until there is no cell suspension remaining in all of the culture containers 10, where the treatment is a treatment in which the transfer control unit 70 extracts a certain amount of the cell suspension corresponding to one unit of treatment in the treatment part 20 from the culture container 10, transfers the amount of the cell suspension to the treatment part 20 to complete the treatment in the treatment part 20, and thereafter, transfers the cell suspension to the mixing part 30.

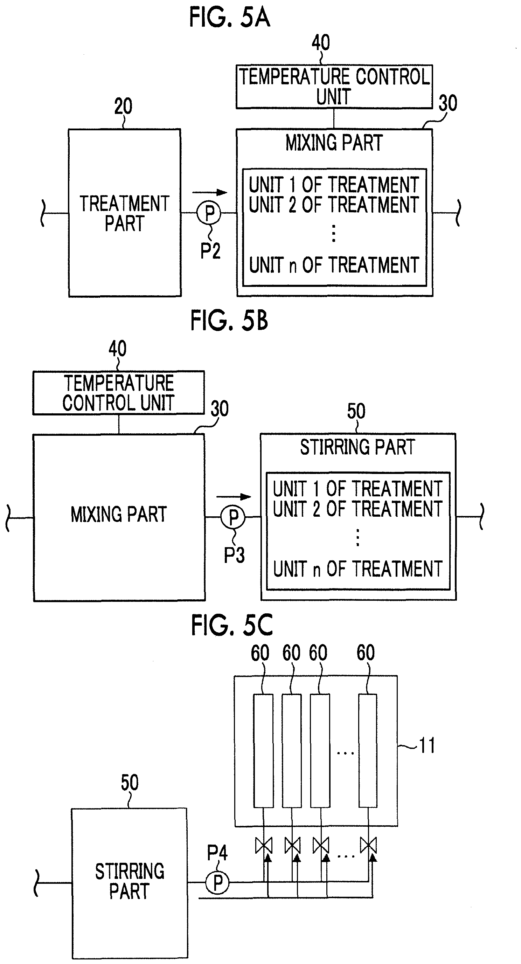

As shown in Fig. 5A, the cell suspension corresponding to each unit of treatment (unit 1 of treatment, unit 2 of treatment, ..., unit n of treatment) which has been treated in the treatment part 20 are accommodated in the mixing part 30. That is, in the mixing part 30, a state in which the cell suspensions corresponding to each unit of treatment are mixed is formed. The term "unit 1 of treatment" corresponds to a cell suspension treated firstly in the treatment part 20, the term "unit 2 of treatment" corresponds to a cell suspension treated secondly in the treatment part 20, and the term "unit n of treatment" corresponds to a cell suspension treated n-thly (lastly) in the treatment part 20. In the present embodiment, the mixing part 30 mixes the cell suspensions treated in the treatment part 20 and corresponding to all of the plurality of units of treatment. It is preferable to inject the cell suspension into the mixing part 30 from a bottom part of the container constituting the mixing part 30. Discharge of air from the mixing part 30 necessary for the injection of the cell suspension into the mixing part 30 is performed via a pipe (not shown) provided at an upper part of the container constituting the mixing part 30. The pipe is preferably provided with a sterile connector, and a flow direction of the air passing through the pipe is preferably always in one direction.

The temperature control unit 40 controls temperature of the cell suspension mixed in the mixing part 30 to a temperature (for example, 37°C) suitable for cell culture. That is, a temperature environment of the cell suspension accommodated in the mixing part 30 may be the same as the temperature environment in the incubator 11. Accordingly, the cell suspension accommodated in the mixing part 30 is kept in a preferable temperature environment suitable for culture until the cell suspension treated n-thly (lastly) is accommodated in the mixing part 30.

In a case where the transfer control unit 70 determines that no cell suspension remains in any of the culture containers 10, the transfer control unit 70 proceeds the treatment to Step S5. In Step S5, the transfer control unit 70 transfers the cell suspension from the mixing part 30 to the stirring part 50 by driving the pump P3. As shown in Fig. 5B, the cell suspension corresponding to each unit of treatment (unit 1 of treatment, unit 2 of treatment, ..., unit n of treatment) is transferred to the stirring part 50. The stirring part 50 stirs the mixture obtained by mixing the cell suspensions corresponding to the respective units of treatment. In the present embodiment, the stirring part 50 stirs the cell suspensions treated in the treatment part 20 and corresponding to all of the plurality of units of treatment.

In Step S6, the transfer control unit 70 determines whether or not the stirring treatment has been completed in the stirring part 50. The transfer control unit 70 may determine that the stirring treatment in the stirring part 50 is completed based on, for example, a state in which a predetermined time has elapsed since the transfer of the cell suspension to the stirring part 50. In addition, in a case where a signal for notifying the completion of stirring treatment is supplied from the stirring part 50, the transfer control unit 70 may determine that the stirring treatment is completed in the stirring part 50 based on the signal. In a case where the transfer control unit 70 determines that the stirring treatment has been completed in the stirring part 50, the treatment proceeds to Step S7. An observation device provided in the stirring part 50 may determine whether or not the number of cells per unit area contained in the cell suspension has been equalized by the stirring treatment in the stirring part 50.

In Step S7, the transfer control unit 70 controls one of the valves V2 to be selectively opened and drives the pump P4. Thereby, the cell suspension stirred in the stirring part 50 is accommodated in the plurality of recovery containers 60. In this case, the cell suspensions may be collectively or sequentially accommodated in the plurality of recovery containers 60. In a case where the cell suspension is sequentially accommodated in the plurality of recovery containers 60, a weight sensor may be provided in each of the recovery containers 60 to determine an opening and closing timing of the valve V2 based on the output of the weight sensor. Fig. 5C illustrates a state in which the cell suspension is accommodated in the plurality of recovery containers 60. Cells contained in the cell suspension accommodated in each of the plurality of recovery containers 60 may be continuously cultured inside the incubator 11. By continuing the culture using the recovery container 60 different from the culture container 10 as a new culture container, the risk of cells being contaminated by bacteria and the like is reduced as compared with a case of reusing the original culture container 10. In a case where the cell suspension is accommodated in each of the plurality of recovery containers 60, controlling a transfer of the cell suspension by the transfer control unit 70 is completed.

In a case where a mass culture of culturing, for example, 10¹⁰ or more cells is performed using the cell culture device 1, there is a concern that changes in treatment states in the treatment part 20 become significant in accordance with an increase in an amount of cell suspensions in cumulative treatment or the number of cells in cumulative treatment in the treatment part 20. For example, in a case where the treatment part 20 includes the filtration device 21, there is a concern that debris such as dead cells accumulates on a filtration membrane in accordance with an increase in an amount of cell suspensions in cumulative treatment, and thus a filtration performance of the filtration membrane changes. In addition, in a case where the treatment part 20 includes the dividing device 22, there is a concern that an aggregate of cells accumulates on the mesh, and thus a membrane surface pressure difference of the mesh change, in accordance with an increase in the number of cells in cumulative treatment. There is a concern that, as a result, a size of the aggregate of the cells after the dividing treatment changes.

As described above, in a case where a mass culture is performed using the cell culture device 1, changes over time in treatment states in the treatment part 20 become significant, and there is a concern that quality of cells treated first in the treatment part 20 becomes different from quality of cells treated subsequently. On the other hand, in a case where cells produced using the cell culture device 1 are used, for example, for regenerative medicine, it is required that quality of cells between the recovery containers 60 be uniform.

According to the cell culture device 1, the cell suspensions corresponding to each of the plurality of units of treatment and treated in the treatment part 20 are mixed in the mixing part 30, and the mixed cell suspensions are stirred in the stirring part 50 and then accommodated in each of the plurality of recovery containers 60. Thereby, the cell suspensions corresponding to each of the plurality of units of treatment (unit 1 of treatment, unit 2 of treatment, ..., unit n of treatment) are accommodated in each of the plurality of recovery containers 60 in a state of being substantially uniformly dispersed. Accordingly, it is possible to inhibit differences in quality of cells between the recovery containers 60. That is, according to the cell culture device 1 according to the embodiment of the disclosed technology, it is possible to absorb a temporal fluctuation in treatment states in the treatment part 20 and to improve uniformity in quality of the cells between the recovery containers 60.

A case in which a total amount of a large amount of cell suspensions is batch-treated in the treatment part will be explained. In this case, a treatment time in the treatment part becomes enormous, and the cell suspension is exposed to a temperature environment that is not suitable for culturing cells for a long time. Therefore, there is a concern that a survival rate of cells or quality of cells is reduced. Accordingly, it is conceivable to keep a temperature of the cell suspension at a temperature approximately equal to a temperature in the incubator (culture temperature) while the treatment is performed in the treatment part. However, for example, during the membrane separation treatment (concentration treatment), a state in which a concentration of the cell suspension is high arises. In this state, in a case where a temperature of the cell suspension is maintained at a temperature approximately equal to a culture temperature, the cells are activated. In the case where the cells are activated, there is a concern that consumption of nutrients and oxygen by the cells is promoted, the nutrients and oxygen are depleted, and thereby the cells are killed. According to the cell culture device 1 according to the embodiment of the disclosed technology, since the temperature control unit 40 controls a temperature of the cell suspension mixed in the mixing part 30, it is possible to prevent the cell suspension from being exposed to a temperature environment unsuitable for culturing cells for a long time without promoting the activation of cells. Thereby, a survival rate of the cells can be increased.

In addition, in a case of culturing while accommodating a total amount of a large amount of cell suspension in a single culture container, a volume of the culture container becomes large. Accordingly, in this case, it becomes difficult to maintain a favorable supply state of oxygen introduced into the culture container through a surface of the culture container to each cell. According to the cell culture device 1 according to the embodiment of the disclosed technology, since the cell suspension is divided and accommodated in the plurality of culture containers 10, a volume of each of the culture containers 10 can be reduced. Thereby, in each of the culture containers 10, it becomes possible to maintain a favorable supply state of oxygen, which is introduced into the culture container 10 through a surface of the culture container 10, to each cell.

The present embodiment exemplifies the case in which the cell suspension is transferred using the pumps P1 to P4 respectively disposed on the flow paths F2 to F5 as a unit for transferring the cell suspension, but the present embodiment is not limited thereto. For example, a transfer of the cell suspension may be performed using pressurizing mechanisms which are respectively provided in each of the culture containers 10, the treatment part 20, the mixing part 30, and the stirring part 50, and which pressurizes a liquid surface of the cell suspension.

### [Second embodiment]

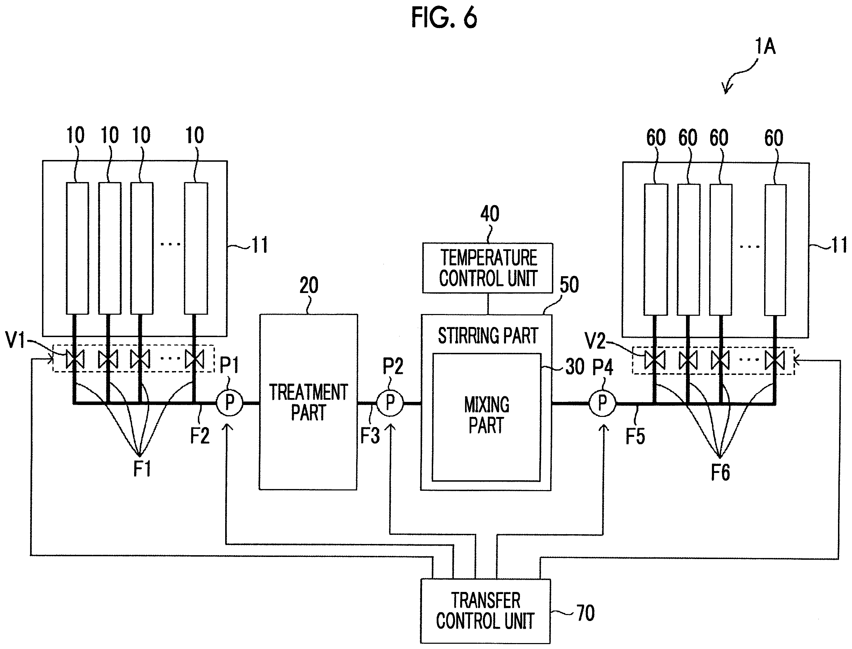

Fig. 6 is a diagram illustrating an example of a configuration of a cell culture device 1A according to a second embodiment of the disclosed technology. The cell culture device 1A is different from the cell culture device 1 (refer to Fig. 1) according to the first embodiment in that the mixing part 30 is configured integrally with the stirring part 50. That is, the mixing part 30 is configured as a treatment container that performs the stirring treatment in the stirring part 50.

The temperature control unit 40 controls a temperature of the cell suspension accommodated in the mixing part 30 functioning as a treatment container of the stirring part 50 to a temperature (for example, 37°C) suitable for culturing cells.

According to the cell culture device 1A according to the second embodiment of the disclosed technology, since the mixing part 30 is configured integrally with the stirring part 50, it is possible to realize a reduction in size of the device. In addition, because it is not necessary to transfer the cell suspension between the mixing part 30 and the stirring part 50, damage to cells due to a transfer of the cell suspension can be reduced.

### [Third embodiment]

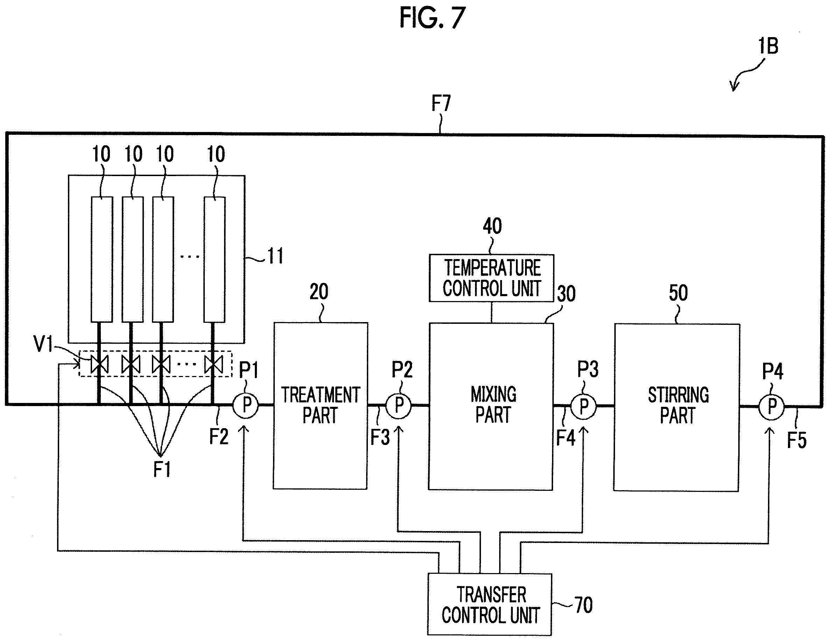

Fig. 7 is a diagram illustrating an example of a configuration of a cell culture device 1B according to a third embodiment of the disclosed technology. The cell culture device 1B is different from the cell culture device 1 (refer to Fig. 1) according to the first embodiment in that the cell culture device 1B includes a flow path F7 for returning the cell suspension stirred in the stirring part 50 to the culture container 10. One end of the flow path F7 is connected to the flow path F5, and the other end of the flow path F7 is connected to the flow path F2.

In the cell culture device 1B, the transfer control unit 70 controls the valve V1 to be opened and drives the pump P4 in Step S7 of the flowchart shown in Fig. 4. Thereby, the cell suspension stirred in the stirring part 50 is accommodated in the plurality of culture containers 10. In this case, the cell suspensions may be collectively or sequentially accommodated in the plurality of culture containers 10. Cells contained in the cell suspension accommodated in each of the plurality of culture containers 10 may be continuously cultured. By continuing the culture by reusing the culture containers 10, it becomes possible to reduce costs as compared with a case of using a new culture container.

### [Fourth embodiment]

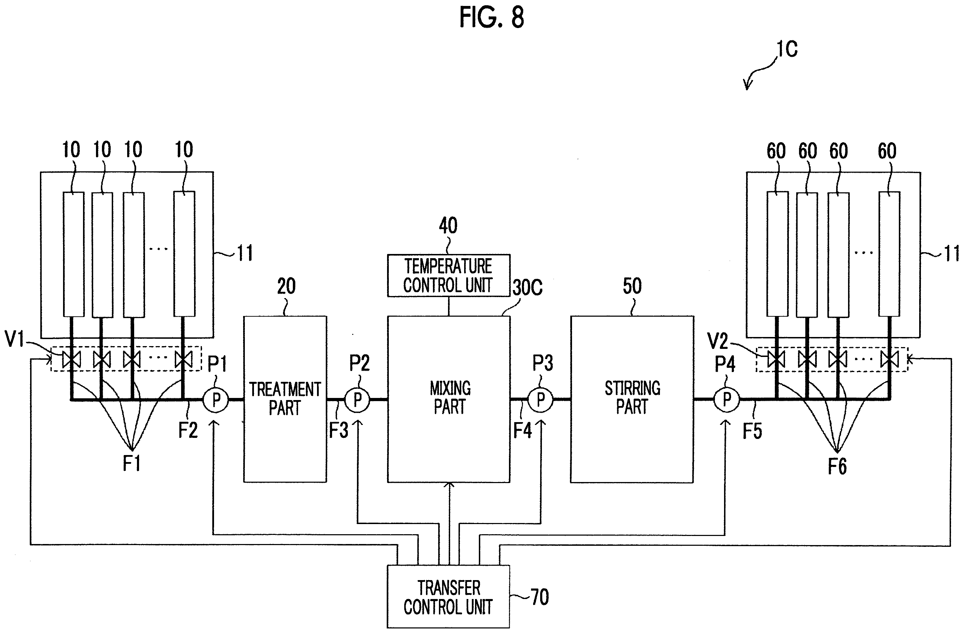

Fig. 8 is a diagram illustrating an example of a configuration of a cell culture device 1C according to a fourth embodiment of the disclosed technology. The cell culture device 1C includes a mixing part 30C.

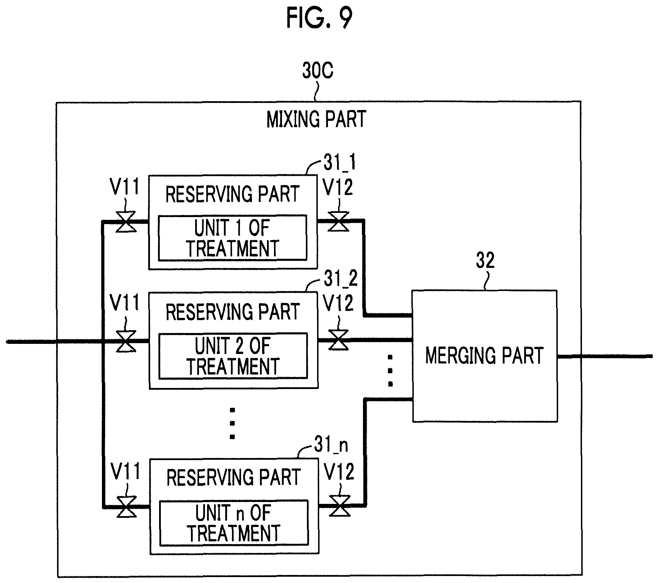

Fig. 9 is a diagram illustrating an example of a configuration of the mixing part 30C. The mixing part 30C includes a plurality of reserving parts 31_1, 31_2, ..., 31_n that reserve the cell suspension treated in the treatment part 20 at each of the units of treatment in the treatment part 20. The reserving part 31_1 reserves the cell suspension corresponding to the "unit 1 of treatment" and treated firstly by the treatment part 20. The reserving part 31_2 reserves the cell suspension corresponding to the "unit 2 of treatment" and treated secondly by the treatment part 20. The reserving part 31_n reserves the cell suspension corresponding to the "unit n of treatment" and treated n-thly (lastly) by the treatment part 20. That is, the cell suspensions respectively corresponding to each of the plurality of units of treatment are reserved in any of the reserving parts 31_1 to 31_n. The temperature control unit 40 controls a temperature of the cell suspension reserved in each of the reserving parts 31_1 to 31_n to a temperature (for example, 37°C) suitable for culturing cells.

The mixing part 30C further includes a merging part 32 that merges the cell suspensions extracted at a predetermined amount from each of the reserving parts 31_1 to 31_n.

Distribution of the cell suspensions to the reserving parts 31_1 to 31_n is performed by controlling opening and closing of a valve V11 provided near each of inlet ports of the reserving parts 31_1 to 31_n. A transfer of the cell suspension from each of the reserving parts 31_1 to 31_n to the merging part 32 is performed by controlling opening and closing of a valve V12 provided near each of outlet ports of the reserving parts 31_1 to 31_n. The controlling opening and closing of the valves V11 and V12 is performed by the transfer control unit 70.

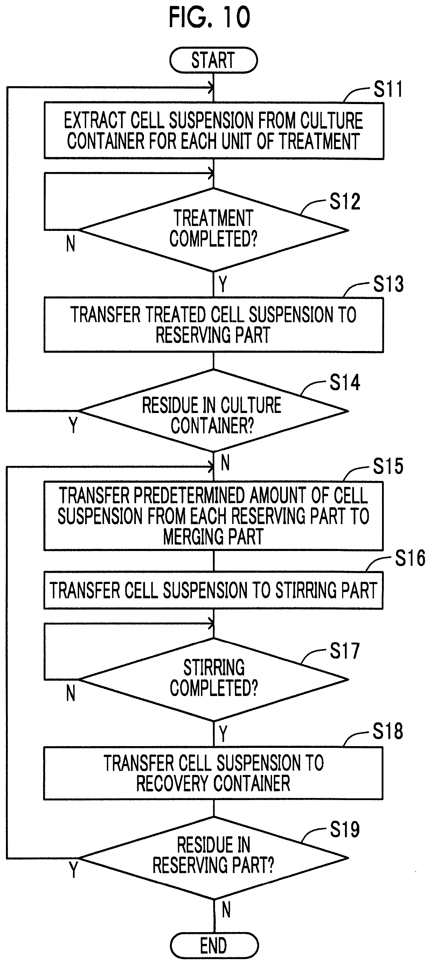

Fig. 10 is a flowchart illustrating an example of an operation sequence of the transfer control unit 70 according to the present embodiment. In an initial state, it is assumed that the cell suspension containing the cells to be cultured is accommodated in each of the plurality of culture containers 10 disposed in the incubator 11.

In Step S11, the transfer control unit 70 controls one of the valves V1 to be selectively opened and drives the pump P1. That is, the transfer control unit 70 extracts a certain amount of the cell suspension corresponding to one unit of treatment in the treatment part 20 among the cell suspensions accommodated in each of the plurality of culture containers 10 from any of the culture containers 10. In this case, the cell suspensions may be extracted from each of the culture containers 10, or the cell suspensions may be extracted from at least one of the culture containers 10. The cell suspension extracted from the culture container 10 is transferred to the treatment part 20 and is subjected to a predetermined treatment in the treatment part 20.

In Step S12, the transfer control unit 70 determines whether the treatment in the treatment part 20 is completed. The transfer control unit 70 may determine that the treatment in the treatment part 20 is completed based on, for example, a state in which a predetermined time has elapsed since the transfer of the cell suspension to the treatment part 20. In addition, in a case where a signal for notifying the completion of treatment is supplied from the treatment part 20, the transfer control unit 70 may determine that the treatment is completed in the treatment part 20 based on the signal. In a case where the transfer control unit 70 determines that the treatment has been completed in the treatment part 20, the treatment proceeds to Step S13.

In Step S13, the transfer control unit 70 controls one of the valves V11 to be selectively opened and drives the pump P2. Accordingly, a certain amount of the cell suspension corresponding to one unit of treatment and treated in the treatment part 20 is transferred to the corresponding reserving part among the reserving parts 31_1 to 31_n.

In Step S14, the transfer control unit 70 determines whether the cell suspension remains in any of the culture containers 10. The transfer control unit 70 may determine whether or not the cell suspension remains in the culture container 10 by, for example, determining whether an accumulated amount of the cell suspension extracted from the culture container 10 has reached a total amount of the cell suspension accommodated in each of the culture containers 10 in the initial state. In addition, in a case where information indicating a weight of the cell suspension accommodated in the culture container 10 is provided in real time, the transfer control unit 70 may determine whether or not the cell suspension remains in the culture container 10 based on the information.

In a case where the transfer control unit 70 determines that the cell suspension remains in any of the culture containers 10, the transfer control unit 70 returns the treatment to Step S11. That is, the transfer control unit 70 repeatedly performs the treatment until there is no cell suspension remaining in the culture container 10, where the treatment is a treatment in which the transfer control unit 70 extracts a certain amount of the cell suspension corresponding to one unit of treatment in the treatment part 20 from the culture container 10, transfers the amount of the cell suspension to the treatment part 20 to complete the treatment in the treatment part 20, and thereafter, transfers the cell suspension to any of the reserving parts 31_1 to 31_n. The cell suspensions treated in the treatment part 20 are respectively reserved in different reserving parts at each of the units of treatment. In a case where the transfer control unit 70 determines that no cell suspension remains in any of the culture containers 10, the transfer control unit 70 proceeds the treatment to Step S15.

In Step S15, the transfer control unit 70 transfers a predetermined amount of the cell suspension from each of the reserving parts 31_1 to 31_n to the merging part 32 by opening all the valves V12 and driving the pump P2. A part of the cell suspensions reserved in each of the reserving parts 31_1 to 31_n is transferred to the merging part 32. The cell suspensions corresponding to each of the units of treatment (unit 1 of treatment, unit 2 of treatment, ..., unit n of treatment) are merged in the merging part 32 to be mixed.

In Step S16, the transfer control unit 70 transfers the cell suspension from the mixing part 30C to the stirring part 50 by driving the pump P3. The cell suspension corresponding to each unit of treatment (unit 1 of treatment, unit 2 of treatment, ..., unit n of treatment) is transferred to the stirring part 50. The stirring part 50 stirs the mixture obtained by mixing the cell suspensions corresponding to the respective units of treatment.

In Step S17, the transfer control unit 70 determines whether or not the stirring treatment has been completed in the stirring part 50. The transfer control unit 70 may determine that the stirring treatment in the stirring part 50 is completed based on, for example, a state in which a predetermined time has elapsed since the transfer of the cell suspension to the stirring part 50. In addition, in a case where a signal for notifying the completion of stirring treatment is supplied from the stirring part 50, the transfer control unit 70 may determine that the stirring treatment is completed in the stirring part 50 based on the signal. In a case where the transfer control unit 70 determines that the stirring treatment has been completed in the stirring part 50, the treatment proceeds to Step S18.

In Step S18, the transfer control unit 70 controls the valve V2 to be opened and drives the pump P4. Thereby, the cell suspension stirred in the stirring part 50 is accommodated in the one recovery container 60 or the plurality of recovery containers 60.

In Step S19, the transfer control unit 70 determines whether the cell suspension remains in each of the reserving parts 31_1 to 31_n. The transfer control unit 70 may determine whether or not the cell suspension remains in the reserving parts 31_1 to 31_n based on, for example, an accumulated amount of the cell suspension extracted from each of the reserving parts 31_1 to 31_n. In addition, in a case where information indicating a weight of the cell suspension reserved in the reserving parts 31_1 to 31_n is provided in real time, the transfer control unit 70 may determine whether or not the cell suspension remains in the reserving parts 31_1 to 31_n based on the information.

In a case where the transfer control unit 70 determines that the cell suspension remains in each of the reserving parts 31_1 to 31_n, the transfer control unit 70 returns the treatment to Step S15. That is, the transfer control unit 70 repeatedly performs the treatment until there is no cell suspension remaining in each of the reserving part 31_1 to 31_n, where the treatment is a treatment in which the transfer control unit 70 extracts a predetermined amount of cell suspension from each of the reserving parts 31_1 to 31_n, transfers the extracted cell suspension to the stirring part 50 via the merging part 32 to complete of the stirring treatment in the stirring part 50, and thereafter, transfers the cell suspension to the recovery containers 60. In a case where the transfer control unit 70 determines that the cell suspensions do not remain in each of the reserving parts 31_1 to 31_n, controlling a transfer of the cell suspension by the transfer control unit 70 is completed.

According to the cell culture device 1C according to the present embodiment, a transfer of the cell suspension from the mixing part 30C to the stirring part 50 is performed in a stepwise manner. Accordingly, the stirring part 50 is not required to have a volume capable of accommodating a total amount of the cell suspensions accommodated in the plurality of culture containers 10. Accordingly, it is possible to reduce a volume of the stirring part 50.

In addition, according to the cell culture device 1C according to the present embodiment, because the cell suspensions reserved in each of the reserving parts 31_1 to 31_n are merged in the merging part 32 to be mixed, even in a case where the stirring part 50 does not have a volume that can accommodate a total amount of the cell suspensions, the cell suspensions corresponding to each of the plurality of units of treatment (unit 1 of treatment, unit 2 of treatment, ..., unit n of treatment) are accommodated in each of the plurality of recovery containers 60 in a state of being substantially uniformly dispersed. Accordingly, it is possible to inhibit differences in quality of cells between the recovery containers 60.

In the cell culture device 1C, the stirring part 50 may be configured integrally with the mixing part 30C according to the example of the cell culture device 1A shown in Fig. 6. In addition, in the cell culture device 1C, the cell suspension stirred in the stirring part 50 may be accommodated in each of the plurality of culture containers 10 according to the example of the cell culture device 1B shown in Fig. 7.

The disclosure of Japanese Patent Application No. 2018-004222 filed on January 15, 2018 is incorporated herein by reference in its entirety. In addition, all documents, patent applications, and technical standards described in the present specification are incorporated herein by reference to the extent in a case where it is specifically and individually stated that individual documents, patent applications, and technical standards are incorporated by reference.

## Claims

1. A cell culture device comprising:
a plurality of culture containers that divide and accommodate a cell suspension;
a treatment part that performs a treatment on a certain amount, which corresponds to each of units of treatment, of at least one of a cell suspension accommodated in each of the plurality of culture containers, or cells contained in the cell suspension accommodated in each of the plurality of culture containers;
a mixing part that mixes cell suspensions which are treated in the treatment part and which respectively correspond to each of the plurality of units of treatment;
a stirring part that stirs the cell suspension mixed in the mixing part; and
a transfer control unit that controls a transfer of the cell suspension to the treatment part, the mixing part, and the stirring part.

2. The cell culture device according to claim 1, further comprising:
a temperature control unit that controls a temperature of the cell suspension mixed in the mixing part.

3. The cell culture device according to claim 1 or 2,
wherein the mixing part is configured integrally with the stirring part.

4. The cell culture device according to any one of claims 1 to 3,
wherein the mixing part mixes the cell suspensions which are treated in the treatment part and which respectively correspond to each of the plurality of units of treatment.

5. The cell culture device according to any one of claims 1 to 4, further comprising:
a plurality of recovery containers that recover the cell suspension stirred in the stirring part,
wherein the transfer control unit further controls a transfer of the cell suspension to each of the plurality of recovery containers.

6. The cell culture device according to any one of claims 1 to 5,
wherein the transfer control unit further controls a transfer of the cell suspension stirred in the stirring part to each of the plurality of culture containers.

7. The cell culture device according to any one of claims 1 to 6,
wherein the mixing part includes
a plurality of reserving parts that reserves the cell suspensions treated in the treatment part or the cell suspensions containing the cells treated in the treatment part for each of the units of treatment in the treatment part, and
a merging part that merges the cell suspensions respectively extracted by a predetermined amount from each of the plurality of reserving parts.

8. The cell culture device according to claim 7,
wherein the cell suspensions, which are treated in the treatment part and respectively correspond to each of the plurality of units of treatment, are respectively reserved in any of the plurality of reserving parts.

9. A cell culture method comprising:
performing a treatment on a certain amount, which corresponds to each of units of treatment, of at least one of a cell suspension or cells contained in the cell suspension;
mixing treated cell suspensions which respectively correspond to each of the plurality of units of treatment; and
stirring the mixed cell suspension.
